# EUROPEAN PATENT SPECIFICATION

(11) **EP 1 363 610 B1**
(45) Date of publication and mention of the grant of the patent: **29.08.2012**
(21) Application number: 02716283.3
(22) Date of filing: 21.01.2002
(51) Int. Cl.: A61K 31/01, A61P 9/12

(54) **CAROTENOIDS AS ANTI-HYPERTENSION AGENTS**
CAROTENOIDE ALS BLUTDRUCKSENKENDES MITTEL
CARETONOIDES UTILISES COMME AGENTS ANTI-HYPERTENSEURS

(30) Priority: 23.01.2001 IL 14103801
(43) Date of publication of application: 26.11.2003
(73) Proprietor: Lycored Natural Products Industries Ltd, 84102 Beer Sheva (IL)
(72) Inventor: HARRIS, Alon, Indeanapolis, IN 46202 (US); ZELKHA, Morris, 84965 Omer (IL); PARAN, Ester, IL-Omer 84965 (IL)
(74) Representative: Modiano, Micaela Nadia
(86) International application number: PCT/IL2002/000054
(87) International publication number: WO 2002/058683

(56) References cited:
- EP-A- 0 759 294
- EP-A- 0 770 385
- WO-A-98/57622
- US-A- 4 046 880
- PATENT ABSTRACTS OF JAPAN vol. 017, no. 489 (C-1106), 6 September 1993 (1993-09-06) & JP 05 124958 A (YUKIO DATE;OTHERS: 01), 21 May 1993 (1993-05-21)
- PATENT ABSTRACTS OF JAPAN vol. 012, no. 213 (C-505), 17 June 1988 (1988-06-17) & JP 63 014678 A (LION CORP), 21 January 1988 (1988-01-21)
- KNEKT P ET AL: "Antioxidant vitamin intake and coronary mortality in a longitudinal population study" AMERICAN JOURNAL OF EPIDEMIOLOGY, SCHOOL OF HYGIENE & PUBLIC HEALTH OF THE JOHNS, US, vol. 139, no. 12, 15 June 1994 (1994-06-15), pages 1180-1189, XP002119579 ISSN: 0002-9262
- KOHLMEIER L ET AL: "LYCOPENE AND MYOCARDIAL INFARCTION RISK IN THE EURAMIC STUDY" , AMERICAN JOURNAL OF EPIDEMIOLOGY, SCHOOL OF HYGIENE & PUBLIC HEALTH OF THE JOHNS, US, VOL. 146, NR. 8, PAGE(S) 618-626 XP000965738 ISSN: 0002-9262 abstract page 624, paragraph DISCUSSION

## Description

### FIELD OF THE INVENTION

The present invention relates to the field of carotenoid containing compositions and uses thereof in reducing blood pressure.

### BACKGROUND OF THE INVENTION

Carotenoids commonly occur in red, yellow, orange and green fruits and vegetables. Carotenoids, either of synthetic or natural sources, are known to be added to food and are taken as nutritional supplements. Lycopene, beta-carotene, phytofluene, phytoene, astaxanthin and cathaxanthin are among the carotenoids which have been found to demonstrate beneficial effects on human health, particularly on the vascular system.

US 5,705,526 (Fujiwara , et al.) relates to a method for treating hypercholesterolemia in a patient in need thereof, which comprises administering to said patient a hypercholesterolemia therapeutic agent containing lycopene as an effective ingredient therein, wherein said lycopene is administered to said patient in an amount within a range of from 1 to 25 mg per day per adult. PCT/IL98/00286 in the name of Lycored Natural Industries Ltd. describes the use of a synergistic mixture of lycopene and vitamin E for inhibiting the oxidation of LDL in human blood, and thus effectively inhibiting the progression of atherosclerosis.

Hypertension, which effects a large number of the population, increases the risk of cardiovascular disease. Thus, there is much interest in methods for reducing blood pressure.

There are at least three known categories of methods for decreasing blood pressure in mammals:
1) Long term therapy on blood lipid profile - Treating atherosclerosis in order to increase the cross-sectional area of the blood vessel. Said treatment is usually via lowering LDL concentration and modifying LDL/HDL ratio in the blood, and preventing/inhibiting the oxidation of LDL in the blood.
2) Treatment of physical properties of the blood (e.g. lowering viscosity and inhibiting/preventing platelet aggregation);
3) Treatment of physical/mechanical properties of blood vessels (e.g. flexibility of the arterial wall and vasodilator response).

Hereinafter said categories referred to as Category 1, Category 2 and Category 3, respectively.

The effect of category 1 treatment on patients is usually noticeable after longer periods of treatment (e.g. more than 2 weeks), whereas treatment under categories 2 and 3 take effect within shorter periods of time (e.g. 2 days to 3 weeks).

Hypercholesterolemia and atherosclerosis which may cause hypertension, are treated according to category 1.

Galley et al, Clinical Science (1997) 92, 361-365, disclose an oral antioxidant synergistic combination comprising beta-carotene, vitamin E, vitamin C and other antioxidants, as effective in lowering blood pressure.
Therapeutic methods within Categories 2 and 3 employ drugs such as aspirin and vasodilating drugs such as nifedipine (hereinafter referred to as "conventional anti-hypertensive agents). There are undesirable side effects associated with the use of said drugs. There is therefore a need for a method for lowering blood pressure which is according to categories 2 and 3, which does have said side effects and is based on natural products.

Thus, it is an objective of the present invention to provide a method for lowering blood pressure by administering natural products, by a method which is not in category 1.

It is a further objective of the present invention to provide a composition for lowering blood pressure by a method which is not in category 1.

Other objectives of the present invention will become apparent as the description proceeds.

### SUMMARY OF THE INVENTION

According to one aspect, the present invention provides a composition for lowering blood pressure in human, wherein said lowering of blood pressure is not achieved by means of treating atherosclerosis, comprising an effective amount of a mixture of carotenoids comprising lycopene, phytofluene and phytoene.
According to another aspect, the present invention relates to the use of a mixture of carotenoids in the preparation of a medicament for lowering blood pressure in a mammal, wherein said lowering of blood pressure is not achieved by means of treating atherosclerosis, and wherein said mixture of carotenoids comprises lycopene, phytofluene and phytoene.
According to another aspect, the present invention relates to a pharmaceutical composition for lowering blood pressure in a mammal, wherein said lowering of blood pressure is not achieved by means of treating atherosclerosis, comprising an effective amount of a mixture of carotenoids, wherein said mixture comprises lycopene, phytofluene, and phytoene.
According to another aspect, the present invention relates to a solid dosage form comprising an effective amount of a mixture of carotenoids useful in lowering blood pressure in a human, wherein said lowering of blood pressure is not achieved by means of treating atherosclerosis, wherein said mixture of carotenoids comprises lycopene, phytofluene and phytoene.

### DETAILED DESCRIPTION OF PREFERRED EMBODIMENTS

The following description is illustrative of embodiments of the invention. The following description is not to be construed as limiting, it being understood that the skilled person may carry out many obvious variations to the invention.

It has unexpectedly been found that the administration of certain carotenoids effect a relatively quick response in the reduction of blood pressure (2 days to 2 weeks).

Throughout the specification the term carotenoids refers to carotenoids of natural, artificial and synthetic sources,

The following embodiments of the invention refer to the lowering of blood pressure not according to Category 1.

About 0.1 to 50 mg per day of the mixture of carotenoids comprising lycopene, phytofluene and phytoene is administered to a subject in need of lowering blood pressure. The administration is preferably oral administration, however, all forms of administration which achieve a blood pressure lowering effective concentration of carotenoid in the blood, e,g. about 0.3 to 0.8 µM of lycopene, is suitable for the purposes of this invention, Administration may be by a single daily dose or multiple doses.

3 to 50 mg of a mixture of carotenoids comprising lycopene (3%-15%), phytoene (0.3%-1%), phytofluene (0.3%-1%) can be administered in order to lower blood pressure in a subject in need thereof. The foregoing mixture may further comprise beta-carotene (0.1%-0,3%) and vitamin E (0,5%-3%).

The composition according to the present invention comprises about 0.1.50 mg of the mixture of carotenoids comprising lycopene, phytofluene and phytoene. The composition may further comprise pharmaceutically acceptable adjuvant, excepients and additives. The composition may be in the form of tablets, capsules, hard shell capsules, gel-caps, soft gels or any further form suitable for the method of administration.

According to a preferred embodiment of the composition, said composition comprises 1 to 5 mg of lycopene.

In yet a further embodiment the composition of the present invention, the composition comprises about 3 to 50 mg of a mixture of carotenoids comprising lycopene (3%-1 5%), phytoene (0.3%-1%), phytofluene (0.3%-1%). The foregoing mixture may further comprise beta-carotene (0.1%-0.3%) and vitamin E (0.5%-3%).

The aforementioned dosages of carotenoids may be administered in conjunction with other conventional anti-hypertension agents. Accordingly, a pharmaceutical composition containing a conventional anti-hypertension agent and the mixture of catenoids comprising lycopene, phytofluene and phytoene is a further embodiment of the present invention.

According to yet a further embodiment of the present invention, the mixture of carotenoids comprising lycopene, phytofluene and phytoene may be added to food stuff, functional foods, dietary supplements and drinks in order to lower blood.

### EXAMPLE

### Example 1

**Design and Methods:** Thirty-five grade I HT subjects, aged 40-65, without concomitant diseases, who required no blood pressure and/or lipid lowering drug therapy, were recruited from primary care clinics. Study participants entered a two weeks run in period for establishment of HT (hypertension) diagnosis and base line evaluation, then 4 weeks placebo and finally 8 weeks treatment periods.
**Results:** Our results demonstrate significant reduction in both systolic BP (blood pressure) from 144 to 135, average of 9 mmHg reduction, and diastolic BP, from 91 to 84, average of 7 mmHg reduction,

### Example 2 (reference)

A double-blind, placebo-controlled crossover study of lycopene consumption in 16 healthy young individuals. Each subject was studied at baseline and then given 15mg lycopene or placebo for two weeks and re-evaluated. A one-month lycopene administration free washout period followed, another lycopene administration was administered prior to a final evaluation. Each evaluation included measures among other parameters also heart rate and blood pressure. During lycopene administration subjects showed a significant reduction in diastolic blood pressure as compared to placebo administration (p<0.05). Table 1 summarizes the results of this study.

**Table 1**

| Physiological parameter | Baseline | Placebo | Lycopene |
|---|---|---|---|
| Systolic Blood Pressure (mmHg) | 112±3 | 111±2 | 114±2 |
| Diastolic Blood Pressure (mmHg) | 70±2 | 70±2 | 66±2 |

While embodiments of the invention have been described by way of illustration, it will be apparent that the invention may be carried out with many modifications, variations and adaptations, without exceeding the scope of the claims,

## Claims

1. Use of a mixture of carotenoids in the preparation of a medicament for lowering blood pressure in a mammal, wherein said lowering of blood pressure is not achieved by means of treating atherosclerosis, and wherein said mixture of carotenoids comprises lycopene, phytofluene and phytoene.

2. Use according to claim I in which said mammal is human.

3. Use according to claim I wherein about 0.1-50 mg of the mixture of carotenoids is applied.

4. Use according to claim 1 wherein the mixture of carotenoids comprises about 1-25 mg of lycopene.

5. Use according to claim 1 wherein the mixture of carotenoids are applied with a conventional anti-hypertensive agent.

6. A composition for lowering blood pressure in a human, wherein said lowering of blood pressure is not achieved by means of treating atherosclerosis, comprising an effective amount of a mixture of carotenoids comprising lycopene, phytofluene and phytoene.

7. A composition according to claim 6 comprising about 0.1 to 50 mg of the mixture of carotenoids and a pharmaceutically acceptable adjuvant, excipient or additive.

8. A composition according to claim 6 comprising about 3% - 15% lycopene, 0.3% - 1% phytoene and 0.3% - 1% phytofluene.

9. A composition according to claim 8 comprising about 3 to 30mg of carotenoid mixture.

10. A composition according to claim 6 wherein said composition is intended for oral administration.

11. A composition according to claim 6 in the form of tablets, capsules, hard shell capsules, gel caps or soft gels.

12. A composition according to claim 6 further comprising a conventional anti-hypertensive agent.

13. A solid dosage form comprising an effective amount of a mixture of carotenoids useful in lowering blood pressure in a human, wherein said lowering of blood pressure is not achieved by means of treating atherosclerosis, wherein said mixture of carotenoids comprises lycopene, phytofluene and phytoene.

14. Use of a composition as described in claim 6 as an additive to food stuff, functional foods, dietary supplements and drinks.

15. A pharmaceutical composition for lowering blood pressure in a mammal, wherein said lowering of blood pressure is not achieved by means of treating atherosclerosis, comprising an effective amount of a mixture of carotenoids, wherein said mixture comprises lycopene, phytofluene, and phytoene.

## Patentansprüche

1. Verwendung einer Mischung aus Carotinoiden in der Herstellung eines Medikaments zur Senkung des Blutdrucks in einem Säugetier, worin die Senkung des Blutdrucks nicht erzielt wird aufgrund von Behandlung von Atherosklerose, und worin die Mischung aus Carotinoiden Lycopin, Phytofluen und Phytoen umfasst.

2. Verwendung gemäß Anspruch 1, in welcher das Säugetier ein Mensch ist.

3. Verwendung gemäß Anspruch 1, worin ungefähr 0,1 bis 50 mg der Mischung aus Carotinoiden angewendet werden.

4. Verwendung gemäß Anspruch 1, worin die Mischung aus Carotinoiden ungefähr 1 bis 25 mg Lycopin umfasst.

5. Verwendung gemäß Anspruch 1, worin die Mischung aus Carotinoiden mit einem konventionellen antihypertensiven Wirkstoff angewendet wird.

6. Eine Zusammensetzung zur Senkung des Blutdrucks in einem Menschen, worin die Senkung des Blutdrucks nicht aufgrund von Behandlung von Atherosklerose erzielt wird, umfassend eine wirksame Menge einer Mischung aus Carotinoiden umfassend Lycopin, Phytofluen und Phytoen.

7. Eine Zusammensetzung gemäß Anspruch 6 umfassend ungefähr 0,1 bis 50 mg der Mischung aus Carotinoiden und einen pharmazeutisch verträglichen Hilfsstoff, ein pharmazeutisch verträgliches Bindemittel oder einen pharmazeutisch verträglichen Zusatzstoff.

8. Eine Zusammensetzung gemäß Anspruch 6 umfassend ungefähr 3% bis 15% Lycopin, 0,3% bis 1% Phytoen und 0,3% bis 1% Phytofluen,

9. Eine Zusammensetzung gemäß Anspruch 8 umfassend ungefähr 3 bis 30 mg an Carotinoid-Mischung.

10. Eine Zusammensetzung gemäß Anspruch 6, worin die Zusammensetzung für die orale Verabreichung beabsichtigt ist.

11. Eine Zusammensetzung gemäß Anspruch 6 in der Form von Tabletten, Kapseln, Hartschalenkapseln, Gelkapseln oder weichen Gelen.

12. Eine Zusammensetzung gemäß Anspruch 6, weiter einen konventionellen antihypertensiven Wirkstoff umfassend.

13. Eine feste Dosierform umfassend eine wirksame Menge einer Mischung aus Carotinoiden, nützlich in der Senkung des Blutdrucks in einem Menschen, worin die Senkung des Blutdrucks nicht aufgrund von Behandlung von Atherosklerose erzielt wird, worin die Mischung aus Carotinoiden Lycopin, Phytofluen und Phytoen umfasst,

14. Verwendung einer Zusammensetzung wie in Anspruch 6 beschrieben als ein Zusatzstoff für Nahrungsmittel, funktionelle Nahrungen, diätetische Ergänzungsmittel und Drinks.

15. Eine pharmazeutische Zusammensetzung zur Senkung des Blutdrucks in einem Säugetier, worin die Senkung des Blutdrucks nicht aufgrund von Behandlung von Atherosklerose erzielt wird, umfassend eine wirksame Menge einer Mischung aus Carotinoiden, worin die Mischung Lycopin, Phytofluen und Phytoen umfasst.

## Revendications

1. Emploi d'un mélange de caroténoïdes dans la préparation d'un médicament pour réduire la pression artérielle d'un mammifère, dans lequel ladite réduction de pression artérielle n'est pas atteinte par le traitement de l'athérosclérose, et dans lequel ledit mélange de caroténoïdes comprend du lycopène, du phytofluène et du phytoène.

2. Emploi selon la revendication 1 dans lequel ledit mammifère est l'homme.

3. Emploi selon la revendication 1 dans lequel environ 0.1-50 mg du mélange de caroténoïdes sont appliqués.

4. Emploi selon la revendication 1 dans lequel le mélange de caroténoides Inclut environ 1-25 mg de lycopène.

5. Emploi selon la revendication 1 dans lequel le mélange de caroténoïdes est appliqué avec un agent anti-hypertenseur conventionnel.

6. Composition pour réduire la pression artérielle chez l'homme, dans laquelle ladite réduction de pression artérielle n'est pas atteinte par le traitement de l'athérosclérose, comprenant une quantité efficace du mélange de caroténoïdes incluant du lycopène, du phytofluène et du phytoène.

7. Composition selon la revendication 6 comprenant environ de 0.1 à 50 mg du mélange de caroténoïdes et un adjuvant, un excipient ou un additif pharmaceutiquement acceptable.

8. Composition selon la revendication 6 comprenant environ 3%-15% de lycopéne, 0.3%-1% de phytoène et 0,3%-1% de phytofluène.

9. Composition selon la revendication 8 comprenant environ 3 à 30mg de mélange de caroténoïdes.

10. Composition selon la revendication 6 dans laquelle ladite composition est destinée à une administration par vole orale.

11. Composition selon la revendication 6 sous forme de comprimés, capsules, capsules à enveloppe dure, capsules gélifiées ou gélules.

12. Composition selon la revendication 6 comprenant en outre un agent anti-hypertenseur conventionnel.

13. Forme de dosage solide comprenant une quantité efficace d'un mélange de caroténoïdes utile pour réduire la pression artérielle chez l'homme, dans laquelle ladite réduction de pression artérielle n'est pas atteinte par le traitement de l'athérosclérose, dans laquelle ledit mélange de caroténoïdes comprend du lycopène, du phytofluène et du phytoène.

14. Emploi d'une composition comme décrite dans la revendication 6 en tant qu'additif pour produits alimentaires, aliments fonctionnels, compléments alimentaires et boisson.

15. Composition pharmaceutique pour réduire la pression artérielle d'un mammifère, dans laquelle ladite réduction de pression artérielle n'est pas atteinte par le traitement de l'athérosclérose, comprenant une quantité efficace d'un mélange de caroténoïdes, dans laquelle ledit mélange comprend du lycopéne, phytofluène, et phytoène.
